# EUROPEAN PATENT APPLICATION

(11) **EP 2 088 149 A1**
(43) Date of publication of application: **12.08.2009**
(21) Application number: 07861039.1
(22) Date of filing: 29.10.2007
(51) Int. Cl.: C07D 471/08, A61K 31/444, A61P 25/28

(54) **PHARMACOLOGICALLY ACTIVE N,N'-SUBSTITUTED 3,7-DIAZABICYCLO Ý3.3.1¨NONANES, PHARMACEUTICAL COMPOSITIONS BASED THEREON AND A METHOD FOR THE USE THEREOF**

(30) Priority: 01.11.2006 RU 2006138456
(71) Applicant: Institute of Physiologically active compunds of the Russian Academy of Sciences (IPAC RAN), Moskovskaya obl., 142432 (RU)
(72) Inventor: BACHURIN, Sergey Olegovich, Moskovskaya obl., 142432 (RU); GRIGOR'EV, Vladimir Viktorovich, Moskovskaya obl., 142432 (RU); ZEFIROV, Nikolay Serafimovich, Moscow, 117421 (RU); LAVROV, Mstislav Igorevich, Moscow, 111672 (RU); LAPTEVA, Vera Leonidovna, Moscow, 119234 (RU); PALYULIN, Vladimir Aleksandrovich, Moscow, 117311 (RU)
(74) Representative: Curtis, Philip Anthony
(86) International application number: PCT/RU2007/000595
(87) International publication number: WO 2008/054252

(57) **Abstract**

N,N'-substituted 3,7-diazabicyclo[3.3.1]nonanes of general formula 1 wherein meanings of radicals correspond to the meanings indicated in the specification, having the property of the both positive allosteric modulators of the AMPA receptors, and potential AMPA receptor blockers, are used for the treatment and prevention of neurodegenerative diseases, and may be particularly used for the treatment of AD (Alzheimer's disease), PD (Parkinson's disease), and other neurodegenerative pathologies. The instant invention also relates to pharmaceutical compositions of compounds 1 and to a method for the treatment of the diseases indicated above.

## Description

The invention generally relates to novel derivatives of N,N'-substituted diazabicyclononanes capable of allosteric modulation of AMPA (2-amino-3-(3-hydroxy-5-methylisoxazol-4-yl)-propionic acid) receptors. More particularly, the instant invention relates to novel pharmacologically active N,N'-substituted 3,7-diazabicyclo[3.3.1]nonanes, and it is useful for the treatment of AD (Alzheimer's disease), PD (Parkinson's disease) and other neurodegenerative diseases. The instant invention also relates to pharmaceutical compositions comprising said compounds. The instant invention also relates to a method for treating the diseases indicated above.

Glutamatergic system to which the AMPA receptors belong, is a main excitating neurotransmitter system in the mammalian brain including humans, and it takes part in realization of a whole series of physiological and pathological processes. A broad range of psycho-neurological diseases such as PD, AD and similar neurodegenerative disorders, is known to be associated with dysregulation of these processes (Doble A. Pharmacology and Therapeutics 1999, V.81, No 3, pp. 163-221).

The AMPA receptors are non-uniformly distributed in the brain. A high concentration of these receptors was found in the superficial layers of novel cortex (neocortex) and in the hippocampus [Monaghan, Brain Res., 1984, V.324, pp.160-164]. In studies on animals and humans, these structures were shown to be mainly responsible for sensorimotor processes and they are a matrix for highly behavioral responses. Thus, due to the AMPA receptors, signaling in cerebral neuro-networks responsible for a combination of cognitive processes is realized.

For the reasons set out above, drugs enhancing the function of the AMPA receptors take part in controlling the processes forming memory as well as the processes responsible for recovery of neurons. In experiments it was shown [Arai, Brain Res., 1992, v.598, p.p.173-184] that enhancement of AMPA-mediated synaptic response increases of induction of a long-term potentiation (LTP). LTP is increase in strength of synaptic contacts which accompanies permanent physiological activity in the brain that is typical during leaning processes. Substances enhancing function of the NMDA receptors promote LTP induction [Granger, Synapse, 1993, V.15, pp.326-329; Arai Brain Res., V.638, pp.343-346].

There is a great body of evidence suggesting that LTP is a physiological memory base. For instance, substances blocking LTP hamper memorizing mechanism in animals and humans [Cerro, Neuroscience, 1992, V.46, pp.1-6].

By now, numerous compounds activating the AMPA receptors have been known, for example, aniracetam [Ito, J. Physiol., 1990, V.424, pap.533-543]. These authors showed that aniracetam enhances synaptic signal on several hippocampus sites though exerting no effect on NMDA-mediated signals [Staubli, 1990, Psychobiology, V.18, pp.377-381; Xiao, Hippocampus, 1991, V.1, pp.373-380]. Aniracetam has properties of "a fast attack", but it is unsuitable for a prolonged use because of lacking durable effect that is a characteristic feature of "behaviorally significant (relevant)" drugs. This drug is efficient only at high concentrations (0.1 mM), and in peripheral use it was shown [Guenzi, J. Chromatogr., 1990, V.530, pp.397-406] to be converted into anisoyl-GABA (about 80% of the drug) which already has no effects similar to aniracetam. Unfortunately, in a majority of cases, compounds with neuroprotective activity either act at high doses or have enhanced toxicity.

Recently, a rather broad class of substances named ampakines^{™} has been discovered. By their physiological effect, these substances are allosteric modulators of the AMPA receptors. These compounds were experimentally shown to be more stable and more efficient than those known earlier [Staubli, PNAS, 1994, V.91: pp.11158-11162].

In view of an extensive development of the investigations related to studying pharmacological effect of similar compounds, intensive ionic current induced by the effect of such allosteric modulators of the AMPA receptors with subsequent depolarization of postsynaptic membrane, has been recently experimentally established to trigger expression mechanism of genes responsible for synthesis of neurotrophins NGF (nerve growth factor) and BDNF (brain-derived neurotrophic factor) - growth factors of neural tissue [Legutko B., Neuropharmacology, 2001, V.40, pp.1019-1027; Ebadi, Neurochemistry International, 2000, V.30, pp.347-374]. Expression process of genes responsible for neurotrophin synthesis is of a great importance in treating neurodegenerative disorders and other psycho-neurological diseases. Thus, BDNF was shown [Siuciak, Brain Research, 1994, V. 633, pp. 326-330] to have antidepressant effect in behavioral models of despair and to decrease blood glucose concentration in mice with diabetes [Ono, J. Biochem. And Bioph. Res. Commun., 1997, Vol. 238, pp. 633-637].

The proposed invention is aimed at solving the task of broadening the arsenal of agents useful as novel efficient allosteric modulators of the AMPA receptors.

Resulting from the studies directed to a search for such compounds including those triggering expression mechanism of genes responsible for synthesis of neurotrophins - growth factors of neural tissue, particularly among the compounds having similar activity, the inventors have discovered a broad group of novel derivatives of N,N'-substituted 3,7-diazabicyclo[3.3.1]nonanes in the form of free bases and salts with pharmacologically acceptable acids, a combined detailed characteristic of which is given below and which represents one of the aspects of the instant invention.

Another aspect of the invention is physiological activity of these compounds manifested by their capability of inducing positive allosteric modulation of the AMPA receptor or antagonistic effect. This will provide a basis for giving consideration to the instant compounds on the one hand as to positive allosteric modulators capable of having cognitive-enhancing properties at different concentrations, and on the other hand, as to potential blockers of the AMPA receptors at higher concentrations.

Additional aspect of the invention consists in providing pharmaceutical compositions comprising from 5 to 30% derivatives of N,N'-substituted 3,7-diazabicyclo[3.3.1]nonanes of general formula I in the form of free bases and salts with pharmacologically acceptable acids and therapeutically acceptable inert auxiliary agents such as carriers, excipients etc., as well as a method for the use thereof.

The technical result of the instant invention is the development of novel derivatives of N,N'-substituted 3,7-diazabicyclo[3.3.1]nonanes in the form of free bases and salts thereof with pharmacologically acceptable acids which compounds are presented by general formula (1): 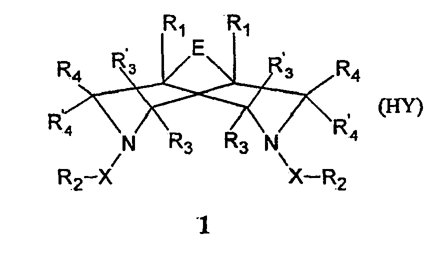 wherein:
HY here and thereafter is a pharmacologically acceptable acid;
E is a carbonyl group, CHR;
R is H, lower alkyl, cycloalkyl, aryl, aralkyl, heteroaryl, hydroxyl, a nitro-group, halogen, alkoxy, acyl, or a group of general formula:

   -(CH₂)ₙ-Q,
wherein n is 1-10, and Q is selected from the group consisting of:
ethenyl optionally mono- or di-substituted by lower alkyl or halogen;
cycloalkyl;
aryl;
heteroaryl;
halogen;
a COOR₅ group, wherein R₅ is H, alkyl, phenyl;
a CH₂OR₅ group, wherein R₅ is as defined above;
a NR₆R₇ group, wherein R₆ and R₇ may be the same or different and each is independently:
H, alkyl, cycloalkyl, aralkyl;
one of substituents of R₆ or R₇ may be a C(O)R₅ group, wherein R₅ is as defined above;
R₁ is H, lower alkyl, cycloalkyl, aryl, aralkyl, heteroaryl, hydroxyl, halogen, alkoxy, acyl, nitro-group, nitrile, or a group of general formula:

   -(CH₂)ₙ-W,
wherein n is as defined above, and W is selected from the group consisting of:
ethenyl optionally mono- or di-substituted by lower alkyl or halogen;
cycloalkyl;
aryl;
heteroaryl;
halogen;
a COOR₈ group, wherein R₈ is H, alkyl, phenyl;
a CH₂OR₈ group, wherein R₈ is as defined above;
a NR₉R₁₀ group, wherein R₉ and R₁₀ may be the same or different and each is independently:
H, alkyl, cycloalkyl, aralkyl;
one of substituents of R₉ or R₁₀ may be a C(O)R₈ group, wherein R₈ is as defined above;
R₂ is presented by general formulas (1.1a), (1.2a), (1.3a), (1.4a): 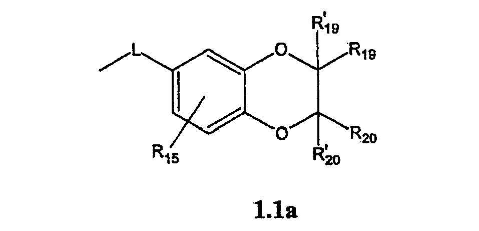 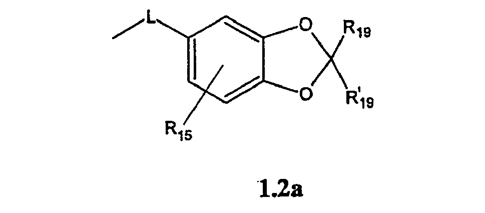 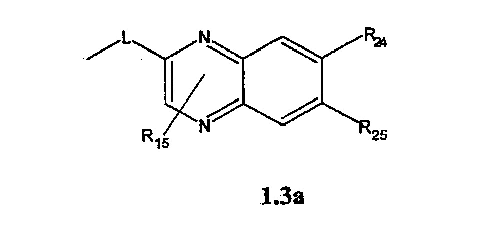 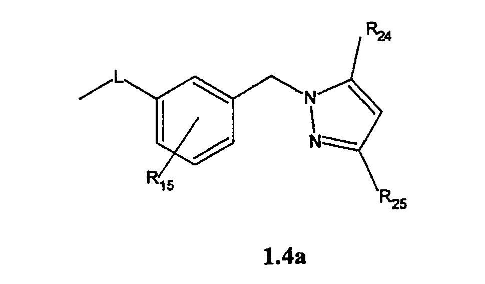
wherein L is CHR₁₁, carbonyl group;
R₁₁ is H, lower alkyl, alkoxy, acyl, cycloalkyl, hydroxyl, halogen, amino or a group of general formula:

   -(CH₂)ₙ-M,
wherein n is as defined above, and M is selected from the group consisting of:
ethenyl optionally mono- or di-substituted by lower alkyl or halogen;
cycloalkyl;
halogen;
a COOR₁₂ group, wherein R₁₂ is H, alkyl;
a CH₂OR₁₂ group, wherein R₁₂ is as defined above;
a NR₁₃R₁₄ group, wherein R₁₃ and R₁₄ may be the same or different and each is independently:
H, alkyl, cycloalkyl, heterocycloalkyl;
one of substituents of R₁₃ or R₁₄ may be C(O)R₁₂ group, wherein R₁₂ is as defined above;
R₁₅ is H, lower alkyl, cycloalkyl, aryl, heteroaryl, hydroxyl, halogen, amino, nitro-group, nitrile, alkoxy, acyl, or a group of general formula:

   - (CH₂)ₙ-U,
wherein n is as defined above, and U is selected from the group consisting of:
ethenyl optionally mono- or di-substituted by lower alkyl or halogen;
cycloalkyl;
aryl;
heteroaryl;
halogen;
a COOR₁₆ group, wherein R₁₆ is H, alkyl, cycloalkyl, heterocycloalkyl;
a CH₂OR₁₆ group, wherein R₁₆ is as defined above;
a NR₁₇R₁₈ group, wherein R₁₇ and R₁₈ may be the same or different and each is independently:
H, alkyl, cycloalkyl, aralkyl, heterocycloalkyl;
one of substituents of R₁₇ or R₁₈ may be C(O)R₁₆ group, wherein R₁₆ is as defined above;
R₁₉, R₁₉', R₂₀ and R₂₀' may be the same or different and each independently is H, lower alkyl, cycloalkyl, aryl, heteroaryl, hydroxyl, halogen, amino, nitro-group, nitrile, alkoxy, acyl, or a group of general formula:

   -(CH₂)ₙ-D,
wherein n is as defined above, and D is selected from the group consisting of:
ethenyl optionally mono- or di-substituted by lower alkyl or halogen;
cycloalkyl;
aryl;
heterocycloalkyl;
heteroaryl;
halogen;
a COOR₂₁ group, wherein R₂₁ is H, alkyl, cycloalkyl, heterocycloalkyl;
a CH₂OR₂₁ group, wherein R₂₁ is as defined above;
a NR₂₂R₂₃ group, wherein R₂₂ and R₂₃ may be the same or different and each is independently:
H, alkyl, cycloalkyl, aralkyl, heterocycloalkyl;
one of substituents of R₂₂ or R₂₃ may be C(O)R₂₁ group, wherein R₂₁ is as defined above;
R₂₄ and R₂₅ may be the same or different and each independently is H, lower alkyl, cycloalkyl, aryl, heteroaryl, hydroxyl, halogen, amino, nitro-group, nitrile, alkoxy, acyl, or a group of general formula:

   -(CH₂)ₙ-A,
wherein n is as defined above, and A is selected from the group consisting of:
ethenyl optionally mono- or di-substituted by lower alkyl or halogen;
cycloalkyl;
aryl;
heterocycloalkyl;
heteroaryl;
halogen;
a COOR₂₆ group, wherein R₂₆ is H, alkyl, cycloalkyl, heterocycloalkyl;
a CH₂OR₂₆ group, wherein R₂₆ is as defined above;
a NR₂₇R₂₈ group, wherein R₂₇ and R₂₈ may be the same or different and each is independently:
H, alkyl, cycloalkyl, aralkyl, heterocycloalkyl;
one of substituents of R₂₇ or R₂₈ may be C(O)R₂₆ group, wherein R₂₆ is as defined above;
in the form of bases or salts thereof with pharmacologically acceptable acids HY;
R₃ and R₃' may be the same or different and each independently is H, lower alkyl, cycloalkyl, aryl, aralkyl, heteroaryl, hydroxyl, halogen, alkoxy, acyl, nitro-group, nitrile, or a group of general formula:

   -(CH₂)ₙ-G,
wherein n is as defined above, and G is selected from the group consisting of:
ethenyl optionally mono- or di-substituted by lower alkyl or halogen;
cycloalkyl;
aryl;
heteroaryl;
halogen;
a COOR₂₉ group, wherein R₂₉ is H, alkyl, phenyl;
a CH₂OR₂₉ group, wherein R₂₉ is as defined above;
a NR₃₀R₃₁ group, wherein R₃₀ and R₃₁ may be the same or different and each is independently:
H, alkyl, cycloalkyl, aralkyl;
one of substituents of R₃₀ or R₃₁ may be C(O)R₂₉ group, wherein R₂₉ is as defined above;
R₄ and R₄' may be the same or different and each independently is H, lower alkyl, cycloalkyl, aryl, aralkyl, heteroaryl, hydroxyl, nitro-group, nitrile, halogen, alkoxy, acyl, or a group of general formula:

   -(CH₂)ₙ-V,
wherein n is as defined above, and V is selected from the group consisting of:
ethenyl optionally mono- or di-substituted by lower alkyl or halogen;
cycloalkyl;
aryl;
heteroaryl;
halogen;
a COOR₃₂ group, wherein R₃₂ is H, alkyl, phenyl;
a CH₂OR₃₂ group, wherein R₃₂ is as defined above;
a NR₃₃R₃₄ group, wherein R₃₃ and R₃₄ may be the same or different and each is independently:
H, alkyl, cycloalkyl, aralkyl;
one of substituents of R₃₃ or R₃₄ may be C(O)R₃₂ group, wherein R₃₂ is as defined above;
X is a group of general formula

   -(CH₂)ₘ-Z-,
wherein m is 0-4, and Z is selected from the group consisting of:
acyl;
iso-alkyl;
acetoxy;
or X is a valence bond.

The term "lower alkyl" as used in the above definitions and subsequent disclosure means an alkyl group with straight or branched chain comprising 1 to 10 carbon atoms, which is exemplified by methyl, ethyl, isopropyl, tert-butyl, isopentyl and similar compounds.

The term "cycloalkyl" means a cyclic saturated hydrocarbon group with 3 to 7 ring carbon atoms; examples thereof include cyclopropyl, cyclopentyl, cyclohexyl and similar compounds.

The term "heterocycloalkyl" means a 4-, 5- or 6-membered N-, O-, or S-heterocycloalkyl ring or substituted analogs thereof. Examples thereof include optionally substituted tetrahydrofuran, tetrahydropyrrole, piperidine etc.

The term "aryl" means unsubstituted or substituted phenyl or naphthyl groups. Phenyl group substituents may be halogens (for example fluoro, chloro, and similar halogens), lower alkyl groups (fro example methyl, ethyl, isopropyl, and similar groups), lower alkoxy groups (for example methoxy, ethoxy, isopropoxy and similar groups). Naphthyl group substituents may be fluoro, chloro, bromo, methyl and methoxy groups.

The term "aralkyl" means the aryl characterized above, to which the alkyl group characterized above is attached.

The term "heteroaryl" means a 5- or 6-membered N-, O-, or S-heteroaromatic ring or a benzoderivative thereof. Examples thereof include optionally substituted furan, thiophene, pyrrole, indole, pyridine, quinoline etc.

Under the term "halogen" as used herein fluoro, chloro, bromo, or iodo are meant.

The term "alkoxy" means an AlkO- group, wherein alkyl moiety is such as an alkyl group defined above. Examples of alkoxy groups include methoxy, butoxy, isopropyloxy and similar groups.

The term "acyl" means a C(O)R group (wherein R is H, alkyl, aryl, and aralkyl as defined above). Examples of acyl groups include formyl, acetyl, benzoyl, phenylacetyl, and similar groups.

The term "amino" means a NR'R" group (wherein R' and R" may be the same or different, and each independently is H, alkyl, cycloalkyl, aryl, aralkyl, heterocycloalkyl, heteroaryl, halogen as defined above). Examples of amino groups include diisopropylamine, diphenylamine, methylethylamine etc.

The term "pharmacologically acceptable acids" encompasses all pharmacologically acceptable acids both inorganic (for example, hydrochloric sulfuric, phosphoric acid, and etc.); and organic acids (for example, formic, acetic, oxalic, citric, tartaric, maleinic, succinic, p-toluene-sulfonic, methylsulfuric acid, etc.).

### Preferable embodiments of the invention

Among the compounds of formula (1), which are one of the subject matters of the instant invention, preferable are the next four groups of compounds which may be represented by formulas (1.1), (1.2), (1.3), and (1.4) set out below. In particular, preferred compounds are:
1.1 N N'- substituted 3,7 - diazabicyclo [3.3.1] nonanes of general formula (1.1): 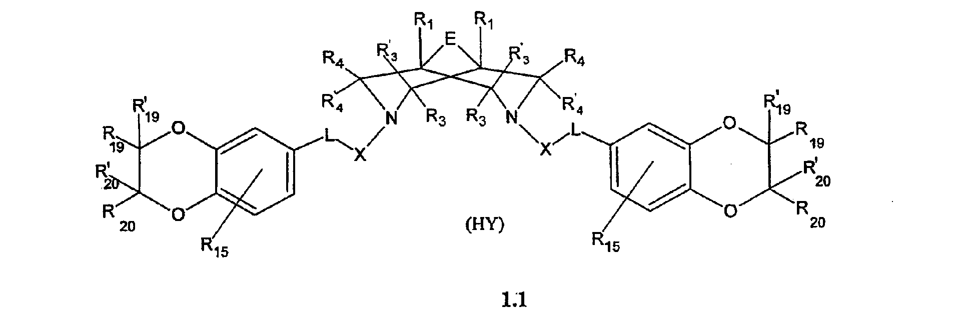
1.2. N,N'-substituted 3,7-diazabicyclo[3.3.1]nonanes of general formula (1.2): 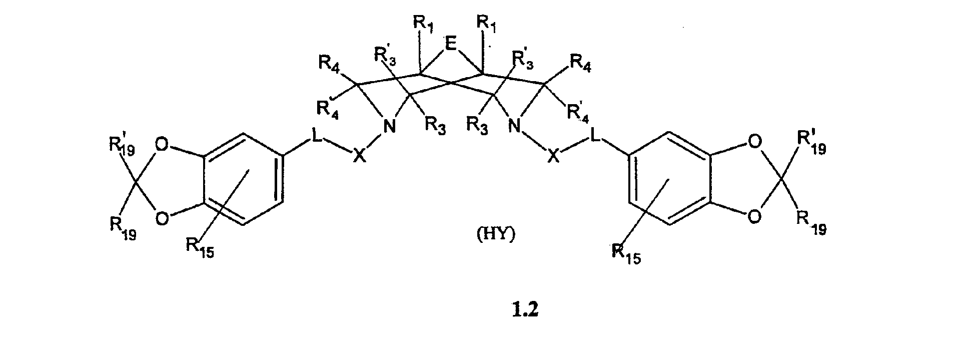
1.2. N,N'-substituted 3,7-diazabicyclo[3.3.1]nonanes of general formula (1.3): 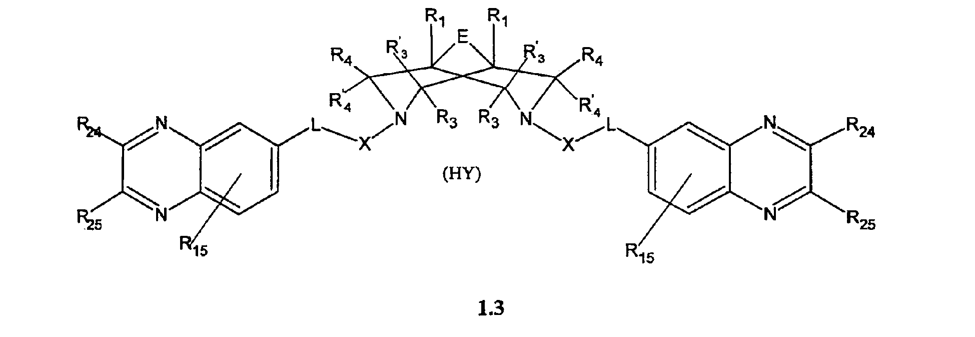
1.2. N,N'-substituted 3,7-diazabicyclo[3.3.1]nonanes of general formula (1.4): 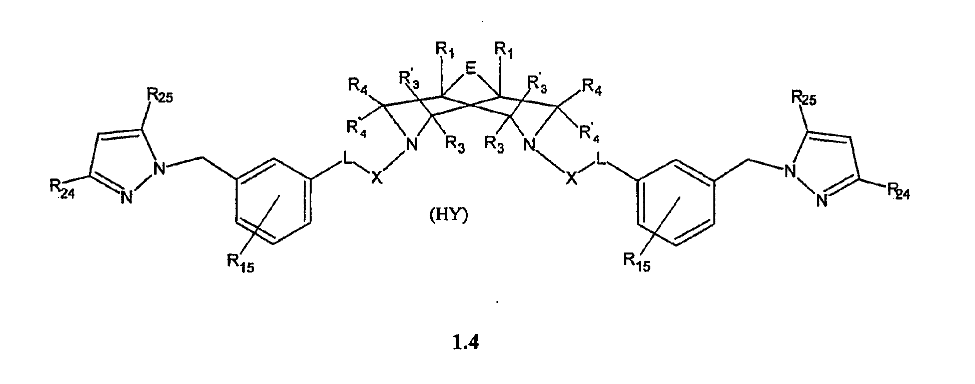
wherein:
HY here and thereafter is a pharmacologically acceptable acid;
E, R₁, R₃, R₃', R₄, R₄' . X, L, R₁₅, R_{19'} R₁₉', R₂₀, R₂₀' , R₂₄ and R₂₅ are as defined above for formula I.

The most preferable compounds of formula 1.1 (in the form of pharmacologically acceptable salts and/or free bases) are:
3,7-bis(2,3-dihydro-1,4-benzodioxin-6-ylcarbonyl)-1,5-dimethyl-3,7-diazabicyclo[3.3.1]nonane
3,7-bis(2,3-dihydro-1,4-benzodioxin-6-ylcarbonyl)-1,5-dimethyl-3,7-diazabicyclo[3.3.1]nonan-9-one
3,7-bis[(7-iodo-2,3-dimethyl-2,3-dihydro-1,4-benzodioxin-6-yl)carbonyl]-1,5-dimethyl-3,7-diazabicyclo[3.3.1]nonan-9-one
3,7-bis[(7-iodo-2,3-dimethyl-2,3-dihydro-1,4-benzodioxin-6-yl)carbonyl]-1,5-dimethyl-3,7-diazabicyclo[3.3.1]nonane
3,7-bis[(3-acetyl-2,3-dihydro-1,4-benzodioxin-6-yl)carbonyl]-1,5-dimethyl-3,7-diazabicyclo[3.3.1]nonan-9-one
3,7-bis[(3-acetyl-2,3-dihydro-1,4-benzodioxin-6-yl)carbonyl]-1,5-dimethyl-3,7-diazabicyclo[3.3.1]nonane
3,7-bis[(2,3-dimethoxy-8-methyl-2,3-dihydro-1,4-benzodioxin-6-yl)carbonyl]-1,5-dimethyl-3,7-diazabicyclo[3.3.1]nonan-9-one
3,7-bis[(2,3-dimethoxy-8-methyl-2,3-dihydro-1,4-benzodioxin-6-yl)carbonyl]-1,5-dimethyl-3,7-diazabicyclo[3.3.1]nonane
3,7-bis{[3-(dimethylamino)-2,3-dihydro-1,4-benzodioxin-6-yl]carbonyl}-1,5-dimethyl-3,7-diazabicyclo[3.3.1]nonan-9-one
3,7-bis{[3-(dimethylamino)-2,3-dihydro-1,4-benzodioxin-6-yl]carbonyl]-1,5-dimethyl-3,7-diazabicyclo[3.3.1]nonane
3,7-bis{[3-(dimethylamino)-8-methyl-2,3-dihydro-1,4-benzodioxin-6-yl]carbonyl}-1,5-dimethyl-3,7-diazabicyclo[3.3.1]nonan-9-one
3,7-bis{[3-(dimethylamino)-8-methyl-2,3-dihydro-1,4-benzodioxin-6-yl]carbonyl}-1,5-dimethyl-3,7-diazabicyclo[3.3.1]nonane
1,5-dimethyl-3,7-bis[(3-nitro-2,3-dihydro-1,4-benzodioxin-6-yl)carbonyl]-3,7-diazabicyclo[3.3.1]nonan-9-one
1,5-dimethyl-3,7-bis[(3-nitro-2,3-dihydro-1,4-benzodioxin-6-yl)carbonyl]-3,7-diazabicyclo[3.3.1]nonane
3,7-bis[(2,3-dicyclopropyl-2,3-dihydro-1,4-benzodioxin-6-yl)carbonyl]-1,5-dimethyl-3,7-diazabicyclo[3.3.1]nonan-9-one
3,7-bis[(2,3-dicyclopropyl-2,3-dihydro-1,4-benzodioxin-6-yl)carbonyl]-1,5-dimethyl-3,7-diazabicyclo[3.3.1]nonane
N,N'-{(1,5-dimethyl-9-oxo-3,7-diazabicyclo[3.3.1]nonan-3,7-diyl)bis[carbonyl(6-iodo-2,3-dihydro-1,4-benzodioxin-7,2-diyl)]}diacetamide
N,N'-{(1,5-dimethyl-3,7-diazabicyclo[3.3.1]nonan-3,7-diyl)bis[carbonyl(6-iodo-2,3-dihydro-1,4-benzodioxin-7,2-diyl)]}diacetamide
dimethyl 7,7'-[(1,5-dimethyl-9-oxo-3,7-diazabicyclo[3.3.1]nonan-3,7-diyl)di(carbonyl)]bis(6-iodo-2,3-dihydro-1,4-benzodioxin-2-carboxylate)
dimethyl 7,7'-[(1,5-dimethyl-3,7-diazabicyclo[3.3.1]nonan-3,7-diyl)di(carbonyl)]bis(6-iodo-2,3-dihydro-1,4-benzodioxin-2-carboxylate)

The most preferable compounds of formula 1.2 (in the form of pharmacologically acceptable salts and/or free bases) are:
1,5-dimethyl-3,7-bis[(2,2,7-trimethyl-1,3-benzodioxol-5-yl)carbonyl]-3,7-diazabicyclo[3.3.1]nonan-9-one
3,7-bis[(4-chloro-2,2,7-trimethyl-1,3-benzodioxol-5-yl)carbonyl]-1,5-dimethyl-3,7-diazabicyclo[3.3.1]nonan-9-one
3,7-bis[(2-acetyl-4-chloro-1,3-benzodioxol-5-yl)carbonyl]-1,5-dimethyl-3,7-diazabicyclo[3.3.1]nonan-9-one
3,7-bis(1,3-benzodioxol-5-ylcarbonyl)-1,5-dimethyl-3,7-diazabicyclo[3.3.1]nonan-9-one
1,5-dimethyl-3,7-bis[(2,2,7-trimethyl-1,3-benzodioxol-5-yl)carbonyl]-3,7-diazabicyclo[3.3.1]nonane
3,7-bis[(4-chloro-2,2,7-trimethyl-1,3-benzodioxol-5-yl)carbonyl]-1,5-dimethyl-3,7-diazabicyclo[3.3.1]nonane
3,7-bis[(2-acetyl-4-chloro-1,3-benzodioxol-5-yl)carbonyl]-1,5-dimethyl-3,7-diazabicyclo[3.3.1]nonane
3,7-bis(1,3-benzodioxol-5-ylcarbonyl)-1,5-dimethyl-3,7-diazabicyclo[3.3.1]nonane
3,7-bis[(7-bromo-2-methoxy-1,3-benzodioxol-5-yl)carbonyl]-1,5-dimethyl-3,7-diazabicyclo[3.3.1]nonan-9-one
3,7-bis[(7-bromo-2-methoxy-1,3-benzodioxol-5-yl)carbonyl]-1,5-dimethyl-3,7-diazabicyclo[3.3.1]nonane
1,5-diethyl-3,7-bis[(6-iodo-2-methoxy-1,3-benzodioxol-5-yl)carbonyl]-3,7-diazabicyclo[3.3.1]nonan-9-one
1,5-diethyl-3,7-bis[(6-iodo-2-methoxy-1,3-benzodioxol-5-yl)carbonyl]-3,7-diazabicyclo[3.3.1]nonane
3,7-bis[(6-iodospiro[1,3-benzodioxol-2,1-cyclohexan]5-yl)carbonyl]-1,5-dimethyl-3,7-diazabicyclo[3.3.1]nonan-9-one
3,7-bis[(6-iodospiro[1,3-benzodioxol-2,1-cyclohexan]5-yl)carbonyl]-1,5-dimethyl-3,7-diazabicyclo[3.3.1]nonane
3,7-bis[(6-iodo-2,2-dimethyl-1,3-benzodioxol-5-yl)carbonyl]-1,5-dimethyl-3,7-diazabicyclo[3.3.1]nonan-9-one
3,7-bis[(6-iodo-2,2-dimethyl-1,3-benzodioxol-5-yl)carbonyl]-1,5-dimethyl-3,7-diazabicyclo[3.3.1]nonane
3,7-bis{[(6-iodo-2-(trifluoromethyl)-1,3-benzodioxol-5-yl]carbonyl}-1,5-dimethyl-3,7-diazabicyclo[3.3.1]nonan-9-one
3,7-bis{[(6-iodo-2-(trifluoromethyl)-1,3-benzodioxol-5-yl]carbonyl}-1,5-dimethyl-3,7-diazabicyclo[3.3.1]nonane
3,7-bis[(2-acetyl-1,3-benzodioxol-5-yl)carbonyl]-1,5-dimethyl-3,7-diazabicyclo[3.3.1]nonan-9-one
3,7-bis[(2-acetyl-1,3-benzodioxol-5-yl)carbonyl]-1,5-dimethyl-3,7-diazabicyclo[3.3.1]nonane

The most preferable compounds of formula 1.3 (in the form of pharmacologically acceptable salts and/or free bases) are:
1,5-dimethyl-3,7-bis(quinoxalin-6-ylcarbonyl)-3,7-diazabicyclo[3.3.1]nonan-9-one
1,5-dimethyl-3,7-bis(quinoxalin-6-ylcarbonyl)-3,7-diazabicyclo[3.3.1]nonane
3,7-bis[(2,3-dimethylquinoxalin-6-yl)carbonyl]-1,5-dimethyl-3,7-diazabicyclo[3.3.1]nonan-9-one
3,7-bis[(2,3-dimethylquinoxalin-6-yl)carbonyl]-1,5-dimethyl-3,7-diazabicyclo[3.3.1]nonane
3,7-bis[(2,3-diisopropylquinoxalin-6-yl)carbonyl]-1,5-dimethyl-3,7-diazabicyclo[3.3.1]nonan-9-one
3,7-bis[(2,3-diisopropylquinoxalin-6-yl)carbonyl]-1,5-dimethyl-3,7-diazabicyclo[3.3.1]nonane
3,7-bis[(7-iodo-3-methoxyquinoxalin-6-yl)carbonyl]-1,5-dimethyl-3,7-diazabicyclo[3.3.1]nonan-9-one
3,7-bis[(7-iodo-3-methoxyquinoxalin-6-yl)carbonyl]-1,5-dimethyl-3,7-diazabicyclo[3.3.1]nonane
1,5-dimethyl-3,7-bis[(2-nitroquinoxalin-6-yl)carbonyl]-3,7-diazabicyclo[3.3.1]nonan-9-one
1,5-dimethyl-3,7-bis[(2-nitroquinoxalin-6-yl)carbonyl]-3,7-diazabicyclo[3.3.1]nonane
3,7-bis[(3-bromo-7-methylquinoxalin-6-yl)carbonyl]-1,5-dimethyl-3,7-diazabicyclo[3.3.1]nonan-9-one
3,7-bis[(3-bromo-7-methylquinoxalin-6-yl)carbonyl]-1,5-dimethyl-3,7-diazabicyclo[3.3.1]nonane
N,N'-[(1,5-dimethyl-9-oxo-3,7-diazabicyclo[3.3.1]nonan-3,7-diyl)bis(carbonylquinoxalin-6,2-diyl)]diacetamide
N,N'-[(1,5-dimethyl-3,7-diazabicyclo[3.3.1]nonan-3,7-diyl)bis(carbonylquinoxalin-6,2-diyl)]diacetamide
3,7-bis[(3-cyclopropylquinoxalin-6-yl)carbonyl]-1,5-diethyl-3,7-diazabicyclo[3.3.1]nonan-9-one
3,7-bis[(3-cyclopropylquinoxalin-6-yl)carbonyl]-1,5-diethyl-3,7-diazabicyclo[3.3.1]nonane
3,7-bis[(8-iodo-3-methylquinoxalin-6-yl)carbonyl]-1,5-dimethyl-3,7-diazabicyclo[3.3.1]nonan-9-one
3,7-bis[(8-iodo-3-methylquinoxalin-6-yl)carbonyl]-1,5-dimethyl-3,7-diazabicyclo[3.3.1]nonane
3,7-bis[1-(2-acetylquinoxalin-6-yl)carbonyl]-1,5-dimethyl-3,7-diazabicyclo[3.3.1]nonan-9-one
3,7-bis[1-(2-acetylquinoxalin-6-yl)carbonyl]-1,5-dimethyl-3,7-diazabicyclo[3.3.1]nonane
3,7-bis[1-(2,3-dimethylquinoxalin-6-yl)ethyl]-1,5-dimethyl-3,7-diazabicyclo[3.3.1]nonan-9-one
3,7-bis[1-(2,3-dimethylquinoxalin-6-yl)ethyl]-1,5-dimethyl-3,7-diazabicyclo[3.3.1]nonane

The most preferable compounds of formula 1.4 (in the form of pharmacologically acceptable salts and/or free bases) are:
N,N'-bis{3-[(3,4-dimethyl-1H-pyrazol-1-yl)methyl]-4-methoxybenzyl}-1,5-dimethyl-9-oxo-3,7-diazabicyclo[3.3.1]nonan-3,7-dicarboxamide
N,N'-bis{3-[(3,4-dimethyl-1H-pyrazol-1-yl)methyl]-4-methoxybenzyl}-1,5-dimethyl-3,7-diazabicyclo[3.3.1]nonan-3,7-dicarboxamide
N,N'-bis{3-[(3,4-dimethyl-1H-pyrazol-1-yl)methyl]-4-methoxy-2-methylbenzyl}-1,5-dimethyl-9-oxo-3,7-diazabicyclo[3.3.1]nonan-3,7-dicarboxamide
N,N'-bis{3-[(3,4-dimethyl-1H-pyrazol-1-yl)methyl]-4-methoxy-2-methylbenzyl}-1,5-dimethyl-3,7-diazabicyclo[3.3.1]nonan-3,7-dicarboxamide
N,N'-bis[(4-methoxy-3-(1H-pyrazol-1-ylmethylbenzyl]-1,5-dimethyl-9-oxo-3,7-diazabicyclo[3.3.1]nonan-3,7-dicarboxamide
N,N'-bis[4-methoxy-3-(1H-pyrazol-1-ylmethyl)benzyl]-1,5-dimethyl-3,7-diazabicyclo[3.3.1]nonan-3,7-dicarboxamide
N,N'-bis{3-[(4-iodo-1H-pyrazol-1-yl)methyl]-4-methoxy-2-methylbenzyl}-1,5-dimethyl-9-oxo-3,7-diazabicyclo[3.3.1]nonan-3,7-dicarboxamide
N,N'-bis{3-[(4-iodo-1H-pyrazol-1-yl)methyl-]-4-methoxy-2-methylbenzyl}-1,5-dimethyl-3,7-diazabicyclo[3.3.1]nonan-3,7-dicarboxamide
3,7-bis[1-{{3-[(4-iodo-1H-pyrazol-1-yl)methyl]-4-methoxybenzyl}amino)ethyl]-1,5-dimethyl-3,7-diazabicyclo[3.3.1]nonan-9-one
3,7-bis[1-{{3-[(4-iodo-1H-pyrazol-1-yl)methyl]-4-methoxybenzyl}amino)ethyl]-1,5-dimethyl-3,7-diazabicyclo[3.3.1]nonane
N,N'-bis{3-[{3,4-[(4-dimethyl-1H-pyrazol-1-yl)methyl]-4-methoxy-2-nitrobenzyl}-1,5-dimethyl-9-oxo-3,7-diazabicyclo[3.3.1]nonan-3,7-dicarboxamide
N,N'-bis{3-[{3,4-[(4-dimethyl-1H-pyrazol-1-yl)methyl]-4-methoxy-2-nitrobenzyl}-1,5-dimethyl-3,7-diazabicyclo[3.3.1]nonan-3,7-dicarboxamide
N,N'-bis{3-(acetylamino)-5-[{3,4-[(4-dimethyl-1H-pyrazol-1-yl)methyl]benzyl}-1,5-dimethyl-9-oxo-3,7-diazabicyclo[3.3.1]nonan-3,7-dicarboxamide
N,N'-bis{3-(acetylamino)-5-[{3,4-dimethyl-1H-pyrazol-1-yl)methyl]benzyl}-1,5-dimethyl-3,7-diazabicyclo[3.3.1]nonan-3,7-dicarboxamide
N,N'-bis{4-methoxy-3-[{4-methoxy-1H-pyrazol-1-yl)methyl]benzyl}-1,5-dimethyl-9-oxo-3,7-diazabicyclo[3.3.1]nonan-3,7-dicarboxamide
N,N'-bis{4-methoxy-3-[{4-methoxy-1H-pyrazol-1-yl)methyl]benzyl}-1,5-dimethyl-3,7-diazabicyclo[3.3.1]nonan-3,7-dicarboxamide
1,5-diethyl-N,N'-bis{3-[{3-iodo-1H-pyrazol-1-yl)methyl]-4-methoxybenzyl}-9-oxo-3,7-diazabicyclo[3.3.1]nonan-3,7-dicarboxamide
1,5-diethyl-N,N'-bis{3-[{3-iodo-1H-pyrazol-1-yl)methyl]-4-methoxybenzyl}-3,7-diazabicyclo[3.3.1]nonan-3,7-dicarboxamide.

Below, the invention is described in more details using examples of preparing particular compounds.

The starting reagents as well as the final products are prepared using the methods known in the literature or are commercially available.

Synthesis schemes of the final compounds are presented below: 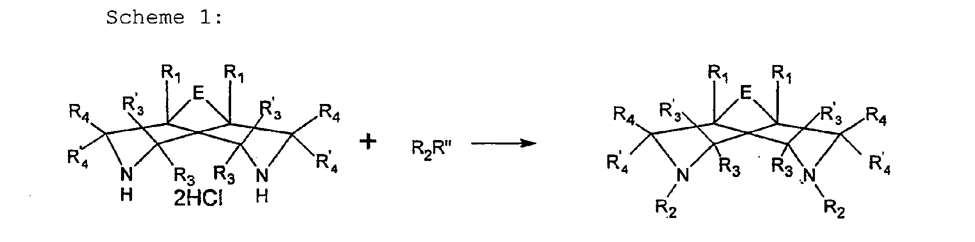 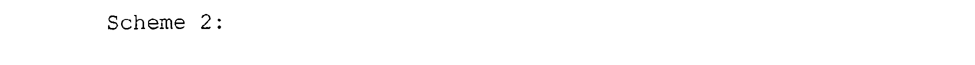 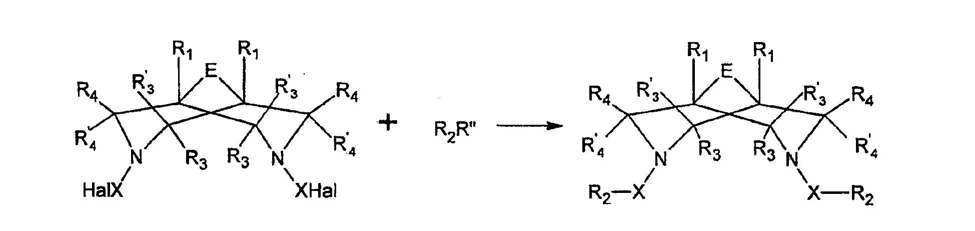 where R" in case of scheme 1 is a halogen or a hydroxy group, and in case of scheme 2 R" is an amino group.

Structures of the prepared compounds were supported by the data of chemical, spectral analyses and other physical-chemical characteristics.

The Examples presented below illustrate but not limit the instant invention.

### Example 1. 3,7-bis(2,3-dihydro-1,4-benzodioxin-6-ylcarbonyl)-1,5-dimethyl-3,7-diazabicyclo[3.3.1]nonan-9-one (compound 1 corresponding to general formula 1.1).

To a solution of 39.8 g (0,2 mole) of chloroanhydride of benzodioxanecarboxylic acide in absolute benzene, 55.2 g (0.4 mole) of finely ground dry potassium carbonate and 24.1 g (0.1 mole) of 1,5-dimethyl-3,7-diazabicyclo[3.3.1]nonan-9-one hydrochloride were added while stirring. The mixture was heated at reflux for six hours. Thereafter, precipitate was filtered off. The filtrate was evaporated to dryness. The residue was applied on a chromatographic silica gel column. Chlorophorm was used as an eluent. A fraction with R_{f}=0.43 was recovered on silufol in the CHCl₃-EtOH (50:1) system. Solvent was distilled off, and a clear oil was obtained which for about 30 minutes was boiled in ethyl ether that resulted in crystallization thereof.
Yield: 72%.
¹HNMR (CDCl₃ δ, ppm): 1.00 s(6H); 3.00 d (2H, J 12 Hz); 3.34 d(2H, J 12 Hz); 4.22 d (2H, J 12 Hz); 4.84d (2H, J 12 Hz); 4.3 s (8H); aromatic protons [7.06 dd (2H, J^{2,1} 8.4 Hz, J^{2,3} 2.4 Hz); 7.09 d (2H, J^{3,2} 2.4 Hz)].

### Example 2. 3,7-bis(1,3-benzodioxol-5-ylcarbonyl)-1,5-dimethyl-3,7-diazabicyclo[3.3.1]nonan-9-one (compound 2 corresponding to general formula 1.2).

To a solution of 37.0 g (0.2 mole) of chloroanhydride of piperonylic acide in absolute benzene, 55.2 g (0.4 mole) of finely ground dry potassium carbonate and 24.1 g (0.1 mole) of 1,5-dimethyl-3,7-diazabicyclo[3.3.1]nonan-9-one hydrochloride were added while stirring. The mixture was heated at reflux for seven hours. Thereafter, precipitate was filtered off. The filtrate was evaporated to dryness. The residue was applied on a chromatographic silica gel column. Chlorophorm was used as an eluent. A fraction with R_{f}=0.4 was recovered on silufol in the CHCl₃-EtOH (50:1) system. Solvent was distilled off, and a clear oil was obtained which for about 30 minutes was boiled in ethyl ether. It was then crystallized.
Yield: 69%.
¹HNMR (CDCl₃ δ, ppm): 1.00 s(6H); 3.00 d (2H, J 12 Hz); 3.34 d(2H, J 12 Hz); 4.22 d (2H, J 12 Hz) ; 4.84 d (2H, J 12 Hz); 4.3 s (8H); aromatic protons [7.06 dd (2H, J^{2,1} 8.4 Hz, J^{2,3} 2.4 Hz); 7.09 d (2H, J^{3,2} 2.4 Hz)].

### Example 3. 3,7-bis[(2,3-dimethylquinoxalin-6-yl)carbonyl]-1,5-dimethyl-3,7-diazabicyclo[3.3.1]nonan-9-one (compound 3 corresponding to general formula 1.3).

To a solution of 40.4 g (0.2 mole) of 2,3-dimethylquinoxaline-6-carboxylic acid in absolute dimethylformamide, 35.64 g (0.22 mole) of CDI (N,N'-carbonyldiimidazole) were added while stirring and cooling in an ice bath. Stirring was carried out for eight hours. Then to the reaction mixture a solution of 24.1 g (0,1 mole) of 1,5-dimethyl-3,7-diazabicyclo[3.3.1]nonan-9-one hydrochloride in DBU (1,8-diazobicyclo[5.4.0]undene-7) was added. The reaction was heated up to 50°C and heated for nine hours. Then precipitate was filtered off. Filtrate was evaporated to dryness. The residue was applied on a chromatographic silica gel column. Chlorophorm was used as an eluent. A fraction with R_{f}=0.57 was recovered on silufol in the CHCl₃-EtOH (20:1) system. Solvent was distilled off, and a clear oil was obtained which over time was crystallized.
Yield: 58%.
¹HNMR (CDCl₃ δ, ppm): 0.97 s(6H); 3.00 d (2H, J 12 Hz); 3.34 d(2H, J 12 Hz); 4.22 d (2H, J 12 Hz); 4.84 d (2H, J 12 Hz); aromatic protons [7.80 d (1H, J=5,8 Hz), 8.18 s (1H), 8.20 d (1H, J=5.8 Hz), 8.94 s (2H)].

### Example 4. N,N'-bis[4-methoxy-3-(1H-pyrazol-1-ylmethyl)benzyl]-1,5-dimethyl-9-oxo-3,7-diazabicyclo[3.3.1]nonan-3,7-dicarboxamide (compound 4 corresponding to general formula 1.4).

To a solution of 52 g (0.2 mole) of 4-methoxy-3-(1H-pyrazol-1-ylmethyl)benzoic acid in absolute dimethylformamide 35.64 g (0.22 mole) of CDI were added while stirring and cooling in an ice bath. Stirring was continued for six hours. Thereafter, a solution of 24.1 g (0.1 mole) of 1,5-dimethyl-3,7-diazabicyclo[3.3.1]nonan-9-one hydrochloride in DBU were added to the reaction mixture. The mixture was heated up to 50°C and heating was continued for seven hours. Thereafter, precipitate was filtered off. Filtrate was evaporated to dryness. The residue was applied on a chromatographic silica gel column. Chlorophorm was used as an eluent. A fraction with R_{f}=0.68 was recovered on silufol in the CHCl₃-EtOH (50:1) system. Solvent was distilled off, and acicular white crystals were produced.
Yield: 79%.
¹HNMR (CDCl₃ δ, ppm): 0.97 s(6H); 2.93 d (2H, J 12 Hz); 3.22 d(2H, J 12 Hz); 3.9 s (6H), 4.05 d (2H, J 12 Hz) ; 4.82 d (2H, J 12 Hz); 5,36 (4H); aromatic protons [6.23 s (2H), 6.97 d (2H, J 5.7 Hz), 7.05 s (2H), 7.5 s (4H), 7.62 d (2H, J 5.9 Hz)].

### Example 5. 3,7-bis[(quinoxalin-6-yl)carbonyl]-1,5-dimethyl-3,7-diazabicyclo[3.3.1]nonan-9-one (compound 5 corresponding to general formula 1.3).

To a solution of 34.8 g (0.2 mole) of quinoxaline-6-carboxylic acid in absolute dimethylformamide, 35.64 g (0.22 mole) of CDI were added while stirring and cooling in an ice bath. Stirring was carried out for five hours. Then a solution of 24.1 g (0.1 mole) of 1,5-dimethyl-3,7-diazabicyclo[3.3.1]nonan-9-one hydrochloride in DBU (1,8-diazabicyclo[5.4.0]undene-7) was added to the reaction mixture. The reaction was heated up to 50°C and heated for eight hours. Then precipitate was filtered off. Filtrate was evaporated to dryness. The residue was applied on a chromatographic silica gel column. Chlorophorm was used as an eluent. A fraction with R_{f}=0.52 was recovered on silufol in the CHCl₃-EtOH (20:1) system. Solvent was distilled off, and a clear oil was obtained which over time was crystallized.
Yield: 64%.
¹HNMR (CDCl₃ δ, ppm): 0.97 s(6H); 3.00 d (2H, J 12 Hz); 3.34 d(2H, J 12 Hz); 4.22 d (2H, J 12 Hz); 4.84 d (2H, J 12 Hz); aromatic protons [7.80 d (1H, J=5.8 Hz), 8.18 s (1H), 8.20 d (1H, J=5.8 Hz)].

### Example 6. 3,7-bis{N-[4-methoxy-3-(1H-pyrazol-1-ylmethyl)benzyl]glicyle}-1,5-dimethyl-3,7-diazabicyclo[3.3.1]nonan-9-one

To a solution of 48 g (0,15 mole) of 3,7-bis(chloroacetyl)-1,5-dimethyl-3,7-diazabicyclo[3.3.1]nonan-9-one in absolute dimethylformamide 64 g (0.3 mole) of [4-methoxy-3-(1H-pyrazol-1-ylmethyl)benzyl]amine in DBU were added while stirring. Stirring was carried out for 30 minutes. Then solvent was evaporated by about 2/3 of a total volume. Residue was diluted with five-fold water excess over the solvent and extracted four times with methylene chloride. Extracts were combined, evaporated to dryness and applied on a chromatographic silica gel column. Chlorophorm was used as an eluent. A fraction with R_{f}=0.73 was recovered on silufol in the CHCl₃-EtOH (50:1) system. Solvent was distilled off, and a clear oil was obtained which was recrystallized from diethyl ether.
Yield: 85%.
¹HNMR (CDCl₃ δ, ppm): 0.97 s(6H); 2.93 d (2H, J 12 Hz); 3.22 d(2H, J 12 Hz), 3.5 s (4H); 3.9 s (6H), 4.05 d (2H, J 12 Hz), 4.5 br.s (2H); 4.82 d (2H, J 12 Hz); 4.9 s (4H); 5.3 d (4H); aromatic protons [6.23 s (2H), 6.97 d (2H, J 5.7 Hz), 7.05 s (2H), 7.3 d (4H, J 5.8 Hz), 7.62 d (2H, J 5.9 Hz)].

The following aspect of the invention relates to the compounds of general formula I which are capable of potentiating (positively modulating) glutamate AMPA receptors.

The inventors used determining the potentiating effect of novel compounds on kainate-induced trans-membrane currents in cerebellar Purkinje's neurons of rats for a directed search for compounds capable of potentiating responses of AMPA receptors (and thereby, to cause improvement of memory and cognitive functions).

### A method for the assessment of potentiating AMPA receptors properties of compounds allowing them to exert effect on glutamatergic neurotransmitter system of the CNS

Experiments on assessing the effect of substances on AMPA receptors were carried out using the patch-clamp method on freshly isolated Purkinje's neurons isolated from cerebellum of rats (aged 12 to 15 days). A modified method was used for isolation. 400 to 600 µm thick cerebellar slices were placed into a thermostatic 10 ml chamber. A solution for isolation had the following composition (in mM): NaCl 150.0, KCl 5.0; CaCl₂ 2.0, MgSO₄×7H₂O 2.0, HEPES 10.0, glucose 15.0, pH 7.42. The slices were incubated in this solution for 60 minutes, and thereafter, the solution was replaced by a similar solution comprising pronase (2 mg/ml) and collagenase (1 mg/ml) and incubated for 70 minutes. After washing with the original solution for 20 minutes, the slices were placed into a Petri plate and separated by a mechanic method using a Pasteur pipette. The solutions were continuously blown-through with 100% O₂ at temperature 34°C. Purkinje's neurons were placed into a 0.6 ml working chamber. A process solution had the following composition (in mM): NaCl 150.0, KCl 5.0; CaCl₂ 2.6, MgSO₄×7H₂O 2.0, HEPES 10.0, glucose 15.0, pH 7.36.

Trans-membrane currents were induced by activation of AMPA receptors by applying agonist solutions of these receptors, i.e. cainic acid (CA) using a fast superfusion method. Cainic acid is an agonist of AMPA receptors and it is used for studying properties of AMPA receptors, since AMPA as such causes a too strong desensitization of the receptors, and it is not used in such experiments. Currents were recorded using borosilicate micro electrodes (resistance 1.5 to 2.5 mOhm) filled with a following composition (in mM): KCl 100.0; EGTA 11.0, CaCl₂ 1.0, MgCl₂ 1.0, HEPES 10.0, ATP 5.0, pH 7.2.

The instrument EPC-9 (HEKA, Germany) was used for recoding. Currents were recorded on the PC Pentium-4 hard disc using the Pulse software, also purchased from the HEKA company. Results were processed using the Pulsefit software (HEKA).

Applying KK induced trans-membrane inward currents in Purkinje's neurons. Addition of the compounds of formula I to perfusion solution induced increase in amplitude of the currents. This increase was dependent on a compound, on concentration thereof, on time elapsed following commencement of a compound application.

**Example 1.** Compound 1 at a dose 0.001 µM caused increase in cainate-induced currents by 40 to 50%, at a dose 0.01 µM - by 80%, at a dose 0.1 µM by 0%, and at a dose 1 µM it caused block of the currents by -20 to -40%. Washing for 3 to 5 minutes returned the amplitude of responses to the control value.

**Example 2.** Compound 2 at a dose 0.00001 µM caused increase in cainate-induced currents by 10%, at a dose 0.0001 µM - by 50-90%, at a dose 0.001 µM by 60-140%, at dose 0.01 µM by 0-20%, and at a dose 0.1 µM it caused block of the currents by -20 to - 40%. Washing for 4 to 6 minutes returned the amplitude of responses to the control value.

The results obtained are presented in Table 1.

**Table 1.**

| Activity of the compounds in potentiating AMPA/cainate-induced currents in rat cerebellar Purkinje's neurons. | |
|---|---|
| **No compound** | **Potentiation of cainate-induced currents (%)** |
| **1 (XXXI)** | 0.0001 µM - no potentiation |
| | 0.001 µM - potentiation by 40 to 50%, |
| | 0.01 µM - potentiation by 80% |
| | 0.1 µM - 0%, |
| | 1 µM - blockade by -20 to -40% |
| **2 (XXX2)** | 0.00001 µM -potentiation by 20 to 40% |
| | 0.0001 µM - potentiation by 50 to 90%, |
| | 0.001 µM - potentiation by 60 to 140% |
| | 0.01 µM - potentiation by 0 to 20%, |
| | 0.1 µM - blockade by -20 to -40% |
| **3 (XXX9)** | 0.0001 µM - no potentiation |
| | 0.001 µM - potentiation by 10 to 15% |
| | 0.01 µM - potentiation by 20 to 30%, |
| | 0.1 µM - blockade by -20 to -50% |
| | 1 µM - no potentiation |
| | 10 µM - blockade by 20 to 50% |
| **4** | 0.001 µM - no effect |
| | 0.01 µM - potentiation by 10 to 30%, |
| | 0.1 µM - blockade by -20 to -40% |
| | 1 µM - 0% |
| | 10 µpM - blockade by 15 to 35% |
| **Memantine** | potentiation by 0 to 15% at 30 µM |

As can be seen from the presented table, the compounds of general formula I possess properties of potentiating currents induced by AMPA receptor activation. By the test of potentiation of AMPA responses, activity of the compounds exceed the standard substance Memantine 33.000 to 3.000.000-fold. At the same time, they have no noticeable neurotoxic effect (LD₅₀ is from 90 to 900 mg/kg within the interval of active studies doses that makes them valuable for use in medicine, especially in treating neurodegenerative diseases such as Alzheimer's disease.

### Performing experiment for determining acute toxicity

Acute toxicity of the substances was determined on males of white outbred mice weighing 22 to 26 grams. The substances were administered intraperitoneally as a solution or a suspension in 1% starch solution. Follow-up period was 14 days. Toxicity in the form of LD₅₀ was calculated according to the Litchfield and Wilcoxon method [Litchfield and Wilcoxon, J. Pharmacol. Exp. Ther., 1949, v. 96, pp. 99-114].

Examples of toxicity of the claimed compounds are presented in Table 2.

**Table 2.**

| Acute toxicity of some compounds of formula I. | |
|---|---|
| Numbers of compounds | LD₅₀ mg/kg |
| 1 | 350 |
| 2 | 140 |
| 3 | 90 |
| 4 | 900 |
| Memantine | 1000 |

The tests performed showed that LD₅₀ of the studied compounds was from 90 to 900 mg/kg. In accordance with the toxicity classification of chemical substances, these compounds belong to substances of moderate and low toxicity. While acute toxicity of the instant compounds somewhat exceeds the value of Memantine's acute toxicity, comparison of the therapeutic index parameters determined as the ratio LD₅₀/ED₅₀ (D.N.Plutitsky et al., - Chem. Pharm. J., 1986, No 10, pp. 1209-1231), shows that a majority of the compounds studied have a significant advantage over Memantine.

As is generally accepted in medicine, it is advisable that the compounds of formula I according to the instant invention be used in the form of compositions which respectively constitute the next aspect of the invention.

A pharmaceutical composition according to the invention is prepared using the conventional procedures in the art and includes a pharmaceutically efficient amount (typically from 5 to 30 wt %) of the active agent representing the compound of formula I or a pharmaceutically acceptable salt thereof (hereinafter referred to as "active compound"), in combination with one or more pharmaceutically acceptable auxiliary additives such as diluents, binders, desintegrants, adsorbents, fragrances, flavoring agents. In accordance with the known methods, pharmaceutical compositions may be represented by different liquid or solid forms.

Examples of solid dosage forms include for instance tablets, pills, gelatin capsules and others.

Examples of liquid dosage forms for injections and parenteral administration include solutions, emulsions, suspensions and other.

Compositions are as a rule prepared using standard procedures which contemplate mixing the active compound with a liquid or a finely ground solid carrier.

Compositions according to the invention in the form of tablets comprise 5 to 30% active compounds and a excipient(s) or a carrier(s). As ingredients for tablets, the following agents are used: a) diluents: beet sugar, lactose, glucose, sodium chloride, sorbitol, mannitol, glycol, disubstituted calcium phosphate; b) binders: magnesium aluminum silicate, starch paste, gelatin, tragacanth, methylcellulose, carboxymethylcellulose and polyvinylpyrrolidone; c) desintegrants: dextrose, agar, alginic acid or salts thereof, starch, Tween.

### Example 1.

100 mg tablets each comprising 0.1 mg Compound 2

| | |
|---|---|
| Compound 2 | 0.1 mg |
| Lactose | 54.9 mg |
| Alginic acid | 20.0 mg |
| Citric acid | 5.0 mg |
| Tragacanth | 20 mg |

A tablet may be formed using compression or molding the active compound with one or more additional ingredients,

Compressed tablets were produced on a special machine. The active ingredient in a free form such as powder or granules in amount of 1 g (the substance amount required to produce 10,000 tablets) was stirred with the binder (tragacanth, 200 g), mixed with the diluent (lactose, 549 g), the desintegrant (alginic acid, 200 g) and the flavor (citric acid, 50 g) were added to the mixture.

For gelatin capsules, colorings and stabilizers were additionally used. Tetrazine and indigo were used as colorings; sodium metabisulphite and sodium benzoate may be represented as stabilizers. The proposed gelatin capsules comprise 1 to 20% active ingredient.

### Example 2

500 mg capsules each comprising 0.5 mg Compound 2

| | |
|---|---|
| Compound 2 | 0.5 mg |
| Glycerol | 100.0 mg |
| Sugar syrup | 339.5 mg |
| Meant oil | 40.0 mg |
| Sodium benzoate | 10.0 mg |
| Ascorbic acid | 5.0 mg |
| Tetrazine | 5.0 mg |

5 g of active substance (compound 2) (the amount required to produce 10,000 capsules) was finely ground and mixed in a mixer with glycerol (1000 g) and sugar syrup (3395 g). After stirring, meant oil (400 g), sodium benzoate (100 g), ascorbic acid (50 g), and tetrazine (50 g) were added to the mixture. Gelatin capsules were prepared using a dropping method. This method allows for performing simultaneous drop dosing of a medicament solution and a heated gelatin mass (900 g gelatin) into a cooled vaseline oil. This result in formation of seamless ball-shaped gelatin capsules filled with medicinal mixture which capsules are completely ready for use and comprise 50 mg active substance.

Injection forms of the composition are preferably isotonic solutions or suspensions. The above forms may be sterilized and comprised of additives such as preservatives: sodium metabisulphite, benzoic acid, sodium benzoate, a mixture of methylparaben and propylparaben; stabilizers: apricot gum and acacia gum, dextrin, starch paste, methylcellulose, Tween; salts regulating osmotic pressure (sodium chloride), or buffers. Furthermore, they may comprise other therapeutically useful substances.

### Example 3

2 ml ampules comprising 1 mg compound 1

| | |
|---|---|
| Compound 1 | 1.0 mg |
| Sodium chloride (0.9% solution) | 1.6 ml |
| Benzoic acid | 10.0 mg |
| Methylcellulose | 10.0 mg |
| Meant oil | 0.4 ml |

To produce injection forms, the active compound 1 (1 g; the amount required to produce 1.000 ampules) was finely ground and mixed in a mixer with meant oil (400 ml), then methylcellulose was added (10 g), mixed with 0.9% sodium chloride solution (1.600 ml) and benzoic acid (10 g) was added. The prepared solution was filled into 2 ml ampules and sterilized with steam for 30 minutes.

The next aspect of the invention is a method for treating or preventing neurodegenerative diseases associated with dysfunction of glutamatergic neurotransmission through the effect on AMPA receptors by administering an efficient amount of the compound of general formula I.

A dose of the active component (the compound of formula I or pharmaceutically acceptable salts thereof) prescribed for administering, varies depending on many factors such as age, sex, patient's weight, symptoms and severity of disease, a particular compound being administered, a method of administration, a form of preparation in which the active compound is prescribed.

Typically, a total prescribed dose is 0.1 to 20 mg daily. The total dose may be divided into several doses for taking for example 1 to 4 times daily. In oral administration, total doses of the active substance range from 0.1 to 20 mg/day, preferably, from 0.1 to 10 mg. In parenteral administration, total doses of the active substance range from 0.5 to 20 mg/day, preferably, from 0.5 to 10 mg, and in intravenous injections - from 0.05 to 5.0 mg/day, preferably, from 0.05 to 2.5 mg. Exact dose may be selected by an attending physician.

## Claims

1. N,N'-substituted 3,7-diazabicyclo[3.3.1]nonanes of general formula I: 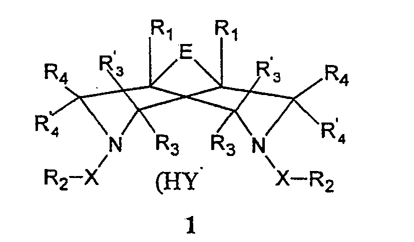 wherein:
HY here and thereafter is a pharmacologically acceptable acid;
E is a carbonyl group, CHR;
R is H, a carbonyl group lower alkyl, cycloalkyl, aryl, aralkyl, heteroaryl, hydroxyl, halogen, alkoxy, acyl, or a group of general formula:
-(CH₂)ₙ-Q,
wherein n is 1-10, and Q is selected from the group consisting of:
ethenyl optionally mono- or di-substituted by lower alkyl or halogen;
cycloalkyl;
aryl;
heteroaryl;
halogen;
a COOR₅ group, wherein R₅ is H, alkyl, phenyl;
a CH₂OR₅ group, wherein R₅ is as defined above;
a NR₆R₇ group, wherein R₆ and R₇ may be the same or different and each is independently H, alkyl, cycloalkyl, aralkyl; one of substituents of R₆ or R₇ may be a C(O)R₅ group, wherein R₅ is as defined above;
R₁ is H, lower alkyl, cycloalkyl, aryl, aralkyl, heteroaryl, hydroxyl, halogen, alkoxy, acyl, or a group of general formula:
-(CH₂)ₙ-W,
wherein n is as defined above, and W is selected from the group consisting of
ethenyl optionally mono- or di-substituted by lower alkyl or halogen;
cycloalkyl;
aryl;
heteroaryl;
halogen;
a COOR₈ group, wherein R₈ is H, alkyl, phenyl;
a CH₂OR₈ group, wherein R₈ is as defined above;
a NR₉R₁₀ group, wherein R₉ and R₁₀ may be the same or different and each is independently:
H, alkyl, cycloalkyl, aralkyl; one of substituents of R₉ or R₁₀ may be a C(O)R₈ group, wherein R₈ is as defined above;
R₂ is presented by general formulas (1.1a), (1.2a), (1.3a), (1.4a): 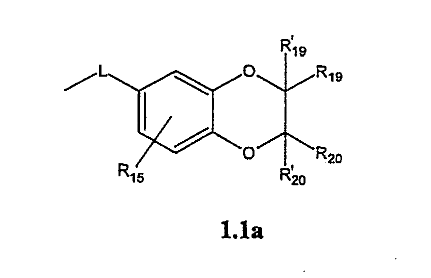 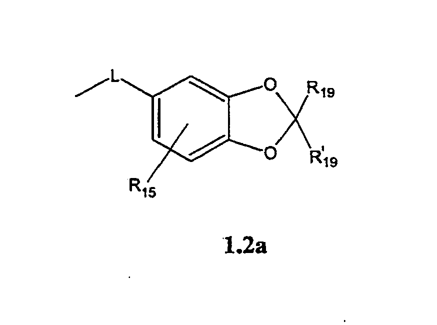 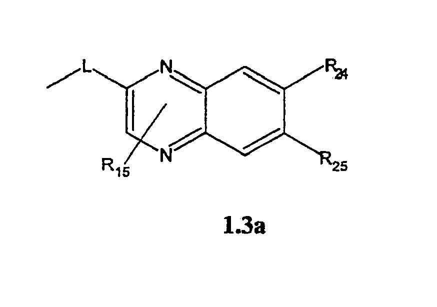 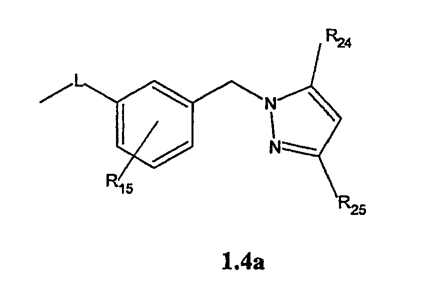
wherein L is CHR₁₁, carbonyl group;
R₁₁ is H, lower alkyl, alkoxy, acyl, cycloalkyl, hydroxyl, halogen, amino or a group of general formula:
-(CH₂)ₙ-M,
wherein n is as defined above, and M is selected from the group consisting of:
ethenyl optionally mono- or di-substituted by lower alkyl or halogen;
cycloalkyl;
halogen;
a COOR₁₂ group, wherein R₁₂ is H, alkyl;
a CH₂OR₁₂ group, wherein R₁₂ is as defined above;
a NR₁₃R₁₄ group, wherein R₁₃ and R₁₄ may be the same or different and each is independently:
H, alkyl, cycloalkyl, heterocycloalkyl;
one of substituents of R₁₃ or R₁₄ may be C(O)R₁₂ group, wherein R₁₂ is as defined above;
R₁₅ is H, lower alkyl, cycloalkyl, aryl, heteroaryl, hydroxyl, halogen, amino, nitro-group, nitrile, alkoxy, acyl, or a group of general formula:
-(CH₂)ₙ-U,
wherein n is as defined above, and U is selected from the group consisting of:
ethenyl optionally mono- or di-substituted by lower alkyl or halogen;
cycloalkyl;
aryl;
heteroaryl;
halogen;
a COOR₁₆ group, wherein R₁₆ is H, alkyl, cycloalkyl, heterocycloalkyl;
a CH₂OR₁₆ group, wherein R₁₆ is as defined above;
a NR₁₇R₁₈ group, wherein R₁₇ and R₁₈ may be the same or different and each is independently:
H, alkyl, cycloalkyl, aralkyl, heterocycloalkyl;
one of substituents of R₁₇ or R₁₈ may be C(O)R₁₆ group, wherein R₁₆ is as defined above;
R₁₉, R₁₉' , R₂₀ and R₂₀' may be the same or different and each is independently H, lower alkyl, cycloalkyl, aryl, heteroaryl, hydroxyl, halogen, amino, nitro-group, nitrile, alkoxy, acyl, or a group of general formula:
-(CH₂)ₙ-D,
wherein n is as defined above, and D is selected from the group consisting of:
ethenyl optionally mono- or di-substituted by lower alkyl or halogen;
cycloalkyl;
aryl;
heterocycloalkyl;
heteroaryl;
halogen;
a COOR₂₁ group, wherein R₂₁ is H, alkyl, cycloalkyl, heterocycloalkyl;
a CH₂OR₂₁ group, wherein R₂₁ is as defined above;
a NR₂₂R₂₃ group, wherein R₂₂ and R₂₃ may be the same or different and each is independently:
H, alkyl, cycloalkyl, aralkyl, heterocycloalkyl;
one of substituents of R₂₂ or R₂₃ may be C(O)R₂₁ group, wherein R₂₁ is as defined above;
R₂₄ and R₂₅ may be the same or different and each is independently H, lower alkyl, cycloalkyl, aryl, heteroaryl, hydroxyl, halogen, amino, nitro-group, nitrile, alkoxy, acyl, or a group of general formula:
-(CH₂)ₙ-A,
wherein n is as defined above, and A is selected from the group consisting of:
ethenyl optionally mono- or di-substituted by lower alkyl or halogen;
cycloalkyl;
aryl;
heterocycloalkyl;
heteroaryl;
halogen;
a COOR₂₆ group, wherein R₂₆ is H, alkyl, cycloalkyl, heterocycloalkyl;
a CH₂OR₂₆ group, wherein R₂₆ is as defined above;
a NR₂₇R₂₈ group, wherein R₂₇ and R₂₈ may be the same or different and each is independently:
H, alkyl, cycloalkyl, aralkyl, heterocycloalkyl;
one of substituents of R₂₇ or R₂₈ may be C(O)R₂₆ group, wherein R₂₆ is as defined above;
in the form of bases or salts thereof with pharmacologically acceptable acids HY;
R₃ and R₃' may be the same or different and each is independently H, lower alkyl, cycloalkyl, aryl, aralkyl, heteroaryl, hydroxyl, halogen, alkoxy, acyl, nitro-group, nitrile, or a group of general formula:
-(CH₂)ₙ-G,
wherein n is as defined above, and G is selected from the group consisting of:
ethenyl optionally mono- or di-substituted by lower alkyl or halogen;
cycloalkyl;
aryl;
heteroaryl;
halogen;
a COOR₂₉ group, wherein R₂₉ is H, alkyl, phenyl;
a CH₂OR₂₉ group, wherein R₂₉ is as defined above;
a NR₃₀R₃₁ group, wherein R₃₀ and R₃₁ may be the same or different and each is independently:
H, alkyl, cycloalkyl, aralkyl;
one of substituents of R₃₀ or R₃₁ may be C(O)R₂₉ group, wherein R₂₉ is as defined above;
R₄ and R₄' may be the same or different and each is independently H, lower alkyl, cycloalkyl, aryl, aralkyl, heteroaryl, hydroxyl, nitro-group, nitrile, halogen, alkoxy, acyl, or a group of general formula:
-(CH₂)ₙ-V,
wherein n is as defined above, and V is selected from the group consisting of:
ethenyl optionally mono- or di-substituted by lower alkyl or halogen;
cycloalkyl;
aryl;
heteroaryl;
halogen;
a COOR₃₂ group, wherein R₃₂ is H, alkyl, phenyl;
a CH₂OR₃₂ group, wherein R₃₂ is as defined above;
a NR₃₃R₃₄ group, wherein R₃₃ and R₃₄ may be the same or different and each is independently:
H, alkyl, cycloalkyl, aralkyl;
one of substituents of R₃₃ or R₃₄ may be C(O)R₃₂ group, wherein R₃₂ is as defined above;
X is a group of general formula
-(CH₂)ₘ-Z-,
wherein m is 0-4, and Z is selected from the group consisting of:
acyl;
iso-alkyl;
acetoxy;
or X is a valence bond.

2. The compounds according to claim 1 representing N,N'-substituted 3,7-diazabicyclo[3.3.1]nonanes of general formula (1.1): 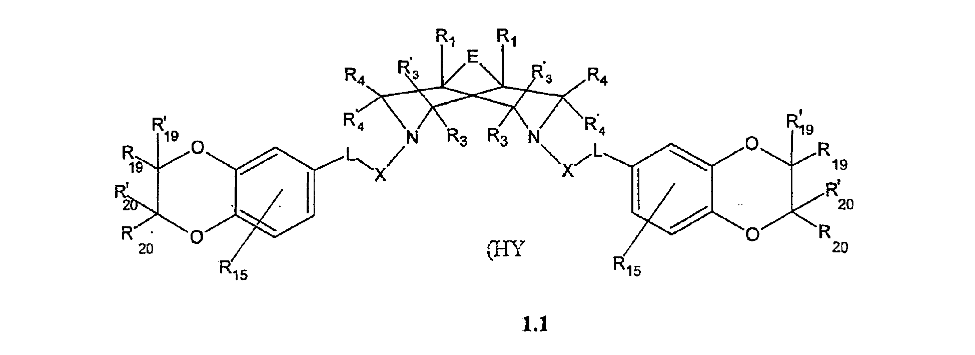 wherein:
HY here and thereafter is a pharmacologically acceptable acid;
E, R₁, R₃, R₃', R₄, R₄', X, L, R₁₅, R₁₉, R₁₉' R₂₀, and R₂₀' are as defined above for formula I.

3. The compounds according to claim 1 representing N,N'-substituted 3,7-diazabicyclo[3.3.1]nonanes of general formula (1.2): 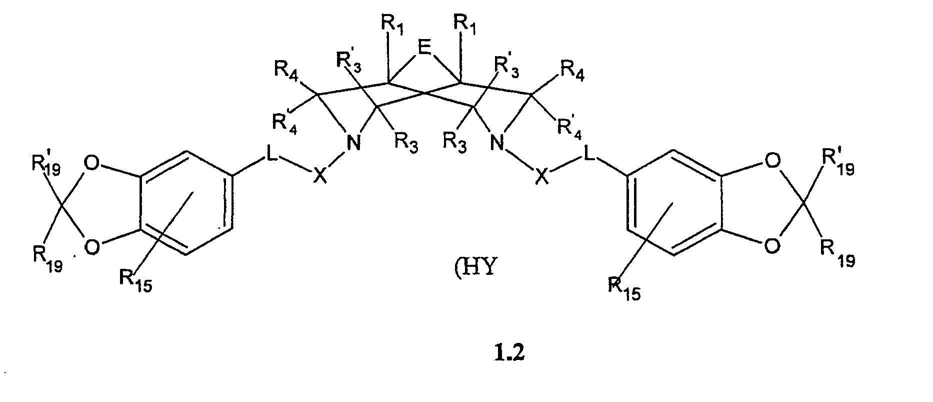 wherein:
HY here and thereafter is a pharmacologically acceptable acid;
E, R₁, R₃, R₃', R₄ R₄', X, L, R₁₅, R₁₉, R₁₉'_{,} R₂₀, and R₂₀' are as defined above for formula I.

4. The compounds according to claim 1 representing N,N'-substituted 3,7-diazabicyclo[3.3.1]nonanes of general formula (1.3): 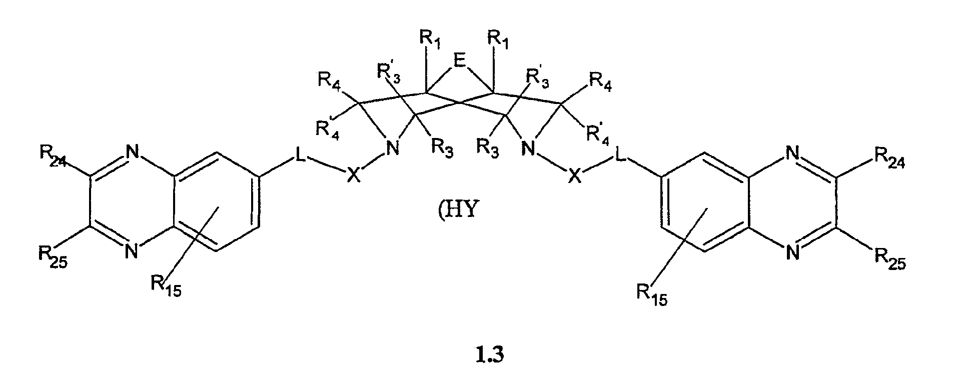 wherein:
HY here and thereafter is a pharmacologically acceptable acid;
E, R₁, R₃, R₃', R₄, R₄' , X, L, R₁₅, R₁₉, R₁₉', R₂₀, R₂₀', R₂₄, and R₂₅, are as defined above for formula I.

5. The compounds according to claim 1 representing N,N'-substituted 3,7-diazabicyclo[3.3.1]nonanes of general formula (1.4): 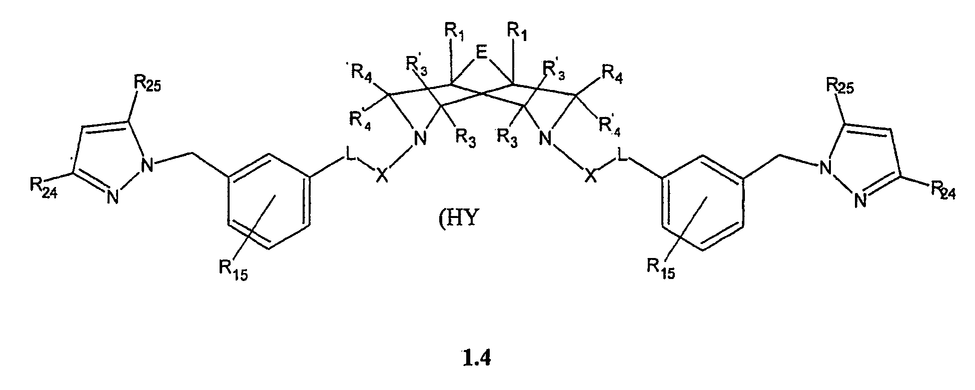 wherein:
HY here and thereafter is a pharmacologically acceptable acid;
E, R₁, R₃, R₃', R₄, R₄', X, L, R₁₅, R₁₉, R₁₉', R₂₀, R₂₀', R₂₄, and R₂₅, are as defined above for formula I.

6. Compounds according to claim 2 representing
3,7-bis(2,3-dihydro-1,4-benzodioxin-6-ylcarbonyl)-1,5-dimethyl-3,7-diazabicyclo[3.3.1]nonane
3,7-bis(2,3-dihydro-1,4-benzodioxin-6-ylcarbonyl)-1,5-dimethyl-3,7-diazabicyclo[3.3.1]nonan-9-one
3,7-bis[(7-iodo-2,3-dimethyl-2,3-dihydro-1,4-benzodioxin-6-yl)carbonyl]-1,5-dimethyl-3,7-diazabicyclo[3.3.1]nonan-9-one
3,7-bis[(7-iodo-2,3-dimethyl-2,3-dihydro-1,4-benzodioxin-6-yl)carbonyl]-1,5-dimethyl-3,7-diazabicyclo[3.3.1]nonane
3,7-bis[(3-acetyl-2,3-dihydro-1,4-benzodioxin-6-yl)carbonyl]-1,5-dimethyl-3,7-diazabicyclo[3.3.1]nonan-9-one
3,7-bis[(3-acetyl-2,3-dihydro-1,4-benzodioxin-6-yl)carbonyl]-1,5-dimethyl-3,7-diazabicyclo[3.3.1]nonane
3,7-bis[(2,3-dimethoxy-8-methyl-2,3-dihydro-1,4-benzodioxin-6-yl)carbonyl]-1,5-dimethyl-3,7-diazabicyclo[3.3.1]nonan-9-one
3,7-bis[(2,3-dimethoxy-8-methyl-2,3-dihydro-1,4-benzodioxin-6-yl)carbonyl]-1,5-dimethyl-3,7-diazabicyclo[3.3.1]nonane
3,7-bis{[3-(dimethylamino)-2,3-dihydro-1,4-benzodioxin-6-yl]carbonyl}-1,5-dimethyl-3,7-diazabicyclo[3.3.1]nonan-9-one
3,7-bis{[3-(dimethylamino)-2,3-dihydro-1,4-benzodioxin-6-yl]carbonyl]-1,5-dimethyl-3,7-diazabicyclo[3.3.1]nonane
3,7-bis{[3-(dimethylamino)-8-methyl-2,3-dihydro-1,4-benzodioxin-6-yl]carbonyl}-1,5-dimethyl-3,7-diazabicyclo[3.3.1]nonan-9-one
3,7-bis{[3-(dimethylamino)-8-methyl-2,3-dihydro-1,4-benzodioxin-6-yl]carbonyl}-1,5-dimethyl-3,7-diazabicyclo[3.3.1]nonane
1,5-dimethyl-3,7-bis[(3-nitro-2,3-dihydro-1,4-benzodioxin-6-yl)carbonyl]-3,7-diazabicyclo[3.3.1]nonan-9-one
1,5-dimethyl-3,7-bis[(3-nitro-2,3-dihydro-1,4-benzodioxin-6-yl)carbonyl]-3,7-diazabicyclo[3.3.1]nonane
3,7-bis[(2,3-dicyclopropyl-2,3-dihydro-1,4-benzodioxin-6-yl)carbonyl]-1,5-dimethyl-3,7-diazabicyclo[3.3.1]nonan-9-one
3,7-bis[(2,3-dicyclopropyl-2,3-dihydro-1,4-benzodioxin-6-yl)carbonyl]-1,5-dimethyl-3,7-diazabicyclo[3.3.1]nonane
N,N'-{(1,5-dimethyl-9-oxo-3,7-diazabicyclo[3.3.1]nonan-3,7-diyl)bis[carbonyl(6-iodo-2,3-dihydro-1,4-benzodioxin-7,2-diyl)]}diacetamide
N,N'-{(1,5-dimethyl-3,7-diazabicyclo[3.3.1]nonan-3,7-diyl)bis[carbonyl(6-iodo-2,3-dihydro-1,4-benzodioxin-7,2-diyl)]}diacetamide
dimethyl 7,7'-[(1,5-dimethyl-9-oxo-3,7-diazabicyclo[3.3.1]nonan-3,7-diyl)di(carbonyl)]bis(6-iodo-2,3-dihydro-1,4-benzodioxin-2-carboxylate)
dimethyl 7,7'-[(1,5-dimethyl-3,7-diazabicyclo[3.3.1]nonan-3,7-diyl)di(carbonyl)]bis(6-iodo-2,3-dihydro-1,4-benzodioxin-2-carboxylate)

7. Compounds according to claim 3 representing
1,5-dimethyl-3,7-bis[(2,2,7-trimethyl-1,3-benzodioxol-5-yl)carbonyl]-3,7-diazabicyclo[3.3.1]nonan-9-one
3,7-bis[(4-chloro-2,2,7-trimethyl-1,3-benzodioxol-5-yl)carbonyl]-1,5-dimethyl-3,7-diazabicyclo[3.3.1]nonan-9-one
3,7-bis[(2-acetyl-4-chloro-1,3-benzodioxol-5-yl)carbonyl]-1,5-dimethyl-3,7-diazabicyclo[3.3.1]nonan-9-one
3,7-bis(1,3-benzodioxol-5-ylcarbonyl)-1,5-dimethyl-3,7-diazabicyclo[3.3.1]nonan-9-one
1,5-dimethyl-3,7-bis[(2,2,7-trimethyl-1,3-benzodioxol-5-yl)carbonyl]-3,7-diazabicyclo[3.3.1]nonane
3,7-bis[(4-chloro-2,2,7-trimethyl-1,3-benzodioxol-5-yl)carbonyl]-1,5-dimethyl-3,7-diazabicyclo[3.3.1]nonane
3,7-bis[(2-acetyl-4-chloro-1,3-benzodioxol-5-yl)carbonyl]-1,5-dimethyl-3,7-diazabicyclo[3.3.1]nonane
3,7-bis(1,3-benzodioxol-5-ylcarbonyl)-1,5-dimethyl-3,7-diazabicyclo[3.3.1]nonane
3,7-bis[(7-bromo-2-methoxy-1,3-benzodioxol-5-yl)carbonyl]-1,5-dimethyl-3,7-diazabicyclo[3.3.1]nonan-9-one
3,7-bis[(7-bromo-2-methoxy-1,3-benzodioxol-5-yl)carbonyl]-1,5-dimethyl-3,7-diazabicyclo[3.3.1]nonane
1,5-diethyl-3,7-bis[(6-iodo-2-methoxy-1,3-benzodioxol-5-yl)carbonyl]-3,7-diazabicyclo[3.3.1]nonan-9-one
1,5-diethyl-3,7-bis[(6-iodo-2-methoxy-1,3-benzodioxol-5-yl)carbonyl]-3,7-diazabicyclo[3.3.1]nonane
3,7-bis[(6-iodospiro[1,3-benzodioxol-2,1-cyclohexan]-5-yl)carbonyl]-1,5-dimethyl-3,7-diazabicyclo[3.3.1]nonan-9-one
3,7-bis[(6-iodospiro[1,3-benzodioxol-2,1-cyclohexan]-5-yl)carbonyl]-1,5-dimethyl-3,7-diazabicyclo[3.3.1]nonane
3,7-bis[(6-iodo-2,2-dimethyl-1,3-benzodioxol-5-yl)carbonyl]-1,5-dimethyl-3,7-diazabicyclo[3.3.1]nonan-9-one
3,7-bis[(6-iodo-2,2-dimethyl-1,3-benzodioxol-5-yl)carbonyl]-1,5-dimethyl-3,7-diazabicyclo[3.3.1]nonane
3,7-bis{[(6-iodo-2-(trifluoromethyl)-1,3-benzodioxol-5-yl]carbonyl}-1,5-dimethyl-3,7-diazabicyclo[3.3.1]nonan-9-one
3,7-bis{[(6-iodo-2-(trifluoromethyl)-1,3-benzodioxol-5-yl]carbonyl}-1,5-dimethyl-3,7-diazabicyclo[3.3.1]nonane
3,7-bis[(2-acetyl-1,3-benzodioxol-5-yl)carbonyl]-1,5-dimethyl-3,7-diazabicyclo[3.3.1]nonan-9-one
3,7-bis[(2-acetyl-1,3-benzodioxol-5-yl)carbonyl]-1,5-dimethyl-3,7-diazabicyclo[3.3.1]nonane.

8. Compounds according to claim 4 representing
1,5-dimethyl-3,7-bis(quinoxalin-6-ylcarbonyl)-3,7-diazabicyclo[3.3.1]nonan-9-one
1,5-dimethyl-3,7-bis(quinoxalin-6-ylcarbonyl)-3,7-diazabicyclo[3.3.1]nonane
3,7-bis[(2,3-dimethylquinoxalin-6-yl)carbonyl]-1,5-dimethyl-3,7-diazabicyclo[3.3.1]nonan-9-one
3,7-bis[(2,3-dimethylquinoxalin-6-yl)carbonyl]-1,5-dimethyl-3,7-diazabicyclo[3.3.1]nonane
3,7-bis[(2,3-diisopropylquinoxalin-6-yl)carbonyl]-1,5-dimethyl-3,7-diazabicyclo[3.3.1]nonan-9-one
3,7-bis[(2,3-diisopropylquinoxalin-6-yl)carbonyl]-1,5-dimethyl-3,7-diazabicyclo[3.3.1]nonane
3,7-bis[(7-iodo-3-methoxyquinoxalin-6-yl)carbonyl]-1,5-dimethyl-3,7-diazabicyclo[3.3.1]nonan-9-one
3,7-bis[(7-iodo-3-methoxyquinoxalin-6-yl)carbonyl]-1,5-dimethyl-3,7-diazabicyclo[3.3.1]nonane
1,5-dimethyl-3,7-bis[(2-nitroquinoxalin-6-yl)carbonyl]-3,7-diazabicyclo[3.3.1]nonan-9-one
1,5-dimethyl-3,7-bis[(2-nitroquinoxalin-6-yl)carbonyl]-3,7-diazabicyclo[3.3.1]nonane
3,7-bis[(3-bromo-7-methylquinoxalin-6-yl)carbonyl]-1,5-dimethyl-3,7-diazabicyclo[3.3.1]nonan-9-one
3,7-bis[(3-bromo-7-methylquinoxalin-6-yl)carbonyl]-1,5-dimethyl-3,7-diazabicyclo[3.3.1]nonane
N,N'-[(1,5-dimethyl-9-oxo-3,7-diazabicyclo[3.3.1]nonan-3,7-diyl)bis(carbonylquinoxalin-6,2-diyl)]diacetamide
N,N'-[(1,5-dimethyl-3,7-diazabicyclo[3.3.1]nonan-3,7-diyl)bis(carbonylquinoxalin-6,2-diyl)]diacetamide
3,7-bis[(3-cyclopropylquinoxalin-6-yl)carbonyl]-1,5-diethyl-3,7-diazabicyclo[3.3.1]nonan-9-one
3,7-bis[(3-cyclopropylquinoxalin-6-yl)carbonyl]-1,5-diethyl-3,7-diazabicyclo[3.3.1]nonane
3,7-bis[(8-iodo-3-methylquinoxalin-6-yl)carbonyl]-1,5-dimethyl-3,7-diazabicyclo[3.3.1]nonan-9-one
3,7-bis[(8-iodo-3-methylquinoxalin-6-yl)carbonyl]-1,5-dimethyl-3,7-diazabicyclo[3.3.1]nonane
3,7-bis[1-(2-acetylquinoxalin-6-yl)carbonyl]-1,5-dimethyl-3,7-diazabicyclo[3.3.1]nonan-9-one
3,7-bis[1-(2-acetylquinoxalin-6-yl)carbonyl]-1,5-dimethyl-3,7-diazabicyclo[3.3.1]nonane
3,7-bis[1-(2,3-dimethylquinoxalin-6-yl)ethyl]-1,5-dimethyl-3,7-diazabicyclo[3.3.1]nonan-9-one
3,7-bis[1-(2,3-dimethylquinoxalin-6-yl)ethyl]-1,5-dimethyl-3,7-diazabicyclo[3.3.1]nonane.

9. Compounds according to claim 5 representing
N,N'-bis{3-[(3,4-dimethyl-1H-pyrazol-1-yl)methyl]-4-methoxybenzyl}-1,5-dimethyl-9-oxo-3,7-diazabicyclo[3.3.1]nonan-3,7-dicarboxamide
N,N'-bis{3-[(3,4-dimethyl-1H-pyrazol-1-yl)methyl]-4-methoxybenzyl}-1,5-dimethyl-3,7-diazabicyclo[3.3.1]nonan-3,7-dicarboxamide
N,N'-bis{3-[(3,4-dimethyl-1H-pyrazol-1-yl)methyl]-4-methoxy-2-methylbenzyl}-1,5-dimethyl-9-oxo-3,7-diazabicyclo[3.3.1]nonan-3,7-dicarboxamide
N,N'-bis{3-[(3,4-dimethyl-1H-pyrazol-1-yl)methyl]-4-methoxy-2-methylbenzyl}-1,5-dimethyl-3,7-diazabicyclo[3.3.1]nonan-3,7-dicarboxamide
N,N'-bis[(4-methoxy-3-(1H-pyrazol-1-ylmethylbenzyl]-1,5-dimethyl-9-oxo-3,7-diazabicyclo[3.3.1]nonan-3,7-dicarboxamide
N,N'-bis[4-methoxy-3-(1H-pyrazol-1-ylmethyl)benzyl]-1,5-dimethyl-3,7-diazabicyclo[3.3.1]nonan-3,7-dicarboxamide
N,N'-bis{3-[(4-iodo-1H-pyrazol-1-yl)methyl]-4-methoxy-2-methylbenzyl}-1,5-dimethyl-9-oxo-3,7-diazabicyclo[3.3.1]nonan-3,7-dicarboxamide
N,N'-bis{3-[(4-iodo-1H-pyrazol-1-yl)methyl]-4-methoxy-2-methylbenzyl}-1,5-dimethyl-3,7-diazabicyclo[3.3.1]nonan-3,7-dicarboxamide
3,7-bis[1-{{3-[(4-iodo-1H-pyrazol-1-yl)methyl]-4-methoxybenzyl}amino)ethyl]-1,5-dimethyl-3,7-diazabicyclo[3.3.1]nonan-9-one
3,7-bis[1-{{3-[(4-iodo-1H-pyrazol-1-yl)methyl]-4-methoxybenzyl}amino)ethyl]-1,5-dimethyl-3,7-diazabicyclo[3.3.1]nonane
N,N'-bis{3-[{3,4-[(4-dimethyl-1H-pyrazol-1-yl)methyl]-4-methoxy-2-nitrobenzyl}-1,5-dimethyl-9-oxo-3,7-diazabicyclo[3.3.1]nonan-3,7-dicarboxamide
N,N'-bis{3-[{3,4-[(4-dimethyl-1H-pyrazol-1-yl)methyl]-4-methoxy-2-nitrobenzyl}-1,5-dimethyl-3,7-diazabicyclo[3.3.1]nonan-3,7-dicarboxamide
N,N'-bis{3-(acetylamino)-5-[{3,4-[(4-dimethyl-1H-pyrazol-1-yl)methyl]benzyl}-1,5-dimethyl-9-oxo-3,7-diazabicyclo[3.3.1]nonan-3,7-dicarboxamide
N,N'-bis{3-(acetylamino)-5-[{3,4-dimethyl-1H-pyrazol-1-yl)methyl]benzyl}-1,5-dimethyl-3,7-diazabicyclo[3.3.1]nonan-3,7-dicarboxamide
N,N'-bis{4-methoxy-3-[{4-methoxy-1H-pyrazol-1-yl)methyl]benzyl}-1,5-dimethyl-9-oxo-3,7-diazabicyclo[3.3.1]nonan-3,7-dicarboxamide
N,N'-bis{4-methoxy-3-[{4-methoxy-1H-pyrazol-1-yl)methyl]benzyl}-1,5-dimethyl-3,7-diazabicyclo[3.3.1]nonan-3,7-dicarboxamide
1,5-diethyl-N,N'-bis{3-[{3-iodo-1H-pyrazol-1-yl)methyl]-4-methoxybenzyl}-9-oxo-3,7-diazabicyclo[3.3.1]nonan-3,7-dicarboxamide
1,5-diethyl-N,N'-bis{3-[{3-iodo-1H-pyrazol-1-yl)methyl]-4-methoxybenzyl}-3,7-diazabicyclo[3.3.1]nonan-3,7-dicarboxamide.

10. The compounds of general formula 1 as defined in any one of claims 1 to 9 having the property of inducing a positive allosteric modulation of AMPA receptor or potential AMPA receptor blockers used for the treatment and prevention of neurodegenerative diseases.

11. A pharmaceutical composition for the treatment and prevention of neurodegenerative diseases comprising an active ingredient and a pharmaceutically acceptable carrier **characterized in that** as the active ingredient it comprises an efficient amount of the compound of general formula I according to any one of claims 1 to 9.

12. A method for treating and preventing neurodegenerative diseases associated with dysfunction of glutamatergic neurotransmission through the effect on AMPA receptors by administering an efficient amount of the compound of general formula I at a dose of from 0.1 to 20 mg daily.
